(19) Europäisches Patentamt
European Patent Office
Office européen des brevets

(11) Publication number: 0 205 981 B1

(12) **EUROPEAN PATENT SPECIFICATION**

(45) Date of publication of patent specification: **02.12.92**

(51) Int. Cl.⁵: **C07G 11/00**, C12P 1/06, C12N 1/20, //(C12P1/06, C12R1:01)

(21) Application number: **86107339.3**

(22) Date of filing: **30.05.86**

# (54) A novel anti-tumor and antimicrobial compound, its microbiological preparation and its use as medicament.

(30) Priority: **12.06.85 JP 128580/85**

(43) Date of publication of application: **30.12.86 Bulletin 86/52**

(45) Publication of the grant of the patent: **02.12.92 Bulletin 92/49**

(84) Designated Contracting States: **AT BE CH DE FR GB IT LI LU NL SE**

(56) References cited:

**THE JOURNAL OF ANTIBIOTICS, vol. XXXV, no. 8, 1982, pages 1013-1019, Japan Antibiotics Res. Ass., Tokyo, JP; S. OMURA et al.: "KITASATOSPORIA, A new genus of the order actinomycetales"**

**THE JOURNAL OF ANTIBIOTICS, vol. XXXVIII, no. 12, 1985, pages 1664-1669, Japan Antibiotics Res. Ass., Tokyo, JP; T. TAMAMURA et al.: "Isolation and characterization of terpentecin, a new antitumor antibiotic"**

(73) Proprietor: **MICROBIAL CHEMISTRY RESEARCH FOUNDATION**
**14-23, Kamiosaki 3 Chome Shinagawa-ku**
**Tokyo 141(JP)**

(72) Inventor: **Umezawa, Hamao, Prof.**
**23, Toyotama-kita 4-chome Nerima-ku**
**Tokyo(JP)**
Inventor: **Takeuchi, Tomio No. 710A, New Fuji Mansion**
**1-11, Higashi-gotanda 5-chome Shinagawa-ku**
**Tokyo(JP)**
Inventor: **Hamada, Masa No. 405, Shuwa Residence**
**26, Naito-cho 1-chome Shinjuku-ku**
**Tokyo(JP)**
Inventor: **Naganawa, Hiroshi**
**17, Denenchofu-honcho 3-chome Ota-ku**
**Tokyo(JP)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 205 981 B1

Inventor: **Takahashi, Yoshikazu**
**2-3, Sakuragaoka 3-chome**
**Tama-city Tokyo(JP)**
Inventor: **Iinuma, Hironobu**
**No. 301, Wako House 35, Shirako 3-chome**
**Wako-city Saitama Prefecture Tokyo(JP)**
Inventor: **Sawa, Tsutomu**
**6-7, Ryosei 4-chome**
**Ayase-city Kanagawa Pref. Tokyo(JP)**
Inventor: **Tamamura, Tsuyoshi**
**2-3, Higashi-rinkan 6-chome**
**Sagamihara-city Kanagawa Pref. Tokyo(JP)**
Inventor: **Isshiki, Kunio 2-13-1332, Shonan Life Town**
**Fujisawa Seibu Danchi 3910, Oba**
**Fujisawa-city Kanagawa Pref. Tokyo(JP)**

(74) Representative: **Müller-Boré & Partner Patentanwälte**
**Isartorplatz 6 Postfach 26 02 47**
**W-8000 München 2(DE)**

## Description

The present invention relates to a novel antiobitic that is produced by a microorganism of the genus Kitasatosporia and which has both anti-tumor and anti-microbial activities.

Compounds of the Anthracycline family (e.g. Adriamycin and Acracinomycin) and many other useful compounds have been proposed as antibiotics having both anti-tumor and anti-microbial activities. However, lots more useful compounds must be provided for the purpose of controlling a wide spectrum of cancers.

While several compounds have been proposed and made known with a view to attaining this object, the substance PR-1350 (Clerocidin) has physicochemical properties closely resembling those of the antibiotic MF 720-N6 according to the present invention (see, for example, Tetrahedron Letters, Vol. 25, No. 4, pp. 465 - 468, 1984). This substance PR-1350 belongs to the family of oxidized diterpenes and is considered to be a complex wherein a monomer containing hydroxyaldehyde of formula (I) and hemiacetal of formula (II) is in equilibrium with a dimer of formula (III) as shown below:

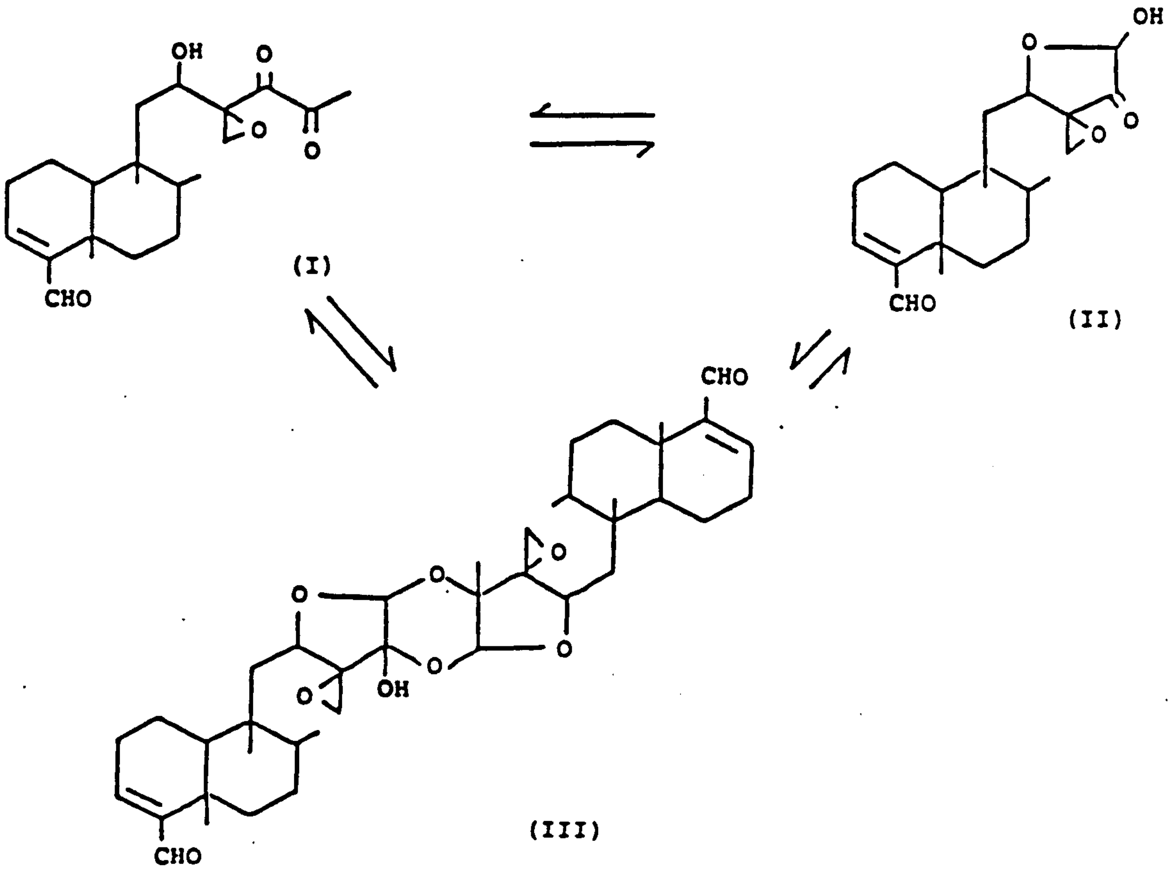

(I)

(II)

(III)

As mentioned above, the prior art has offered many antibiotics of a new family that have both anti-tumor and anti-microbial activities. However, in view of the many types of cancers known today, the demand for the provision of new anti-tumor substances will continue for many years to come.

Now, it has been found that the novel antibiotic MF 730-N6 has strong anti-tumor acitivities both in vitro and in vivo against a variety of tumor cells in laboratory animals and appreciably inhibits the growth of Gram-positive and Gram-negative bacteria.

The antibiotic MF 730-N6 in accordance with the present invention is characterized by the following physicochemical and biological properties:

Physiological properties of antibiotic MF 730-N6

The antibiotic MF 730-N6, like the known PR-1350 substance, is considered to be a complex of tautometric isomers, and hence, will present different physicochemical properties depending upon the conditions of measurement and the method of preparing samples. The physicochemical properties of MF 730-N6 described below are those of a white powder prepared by the method described in Example 1

given later in this specification.

(1) Melting point:

The antibiotic MF 730-N6 generally exhibits a melting point in the range of 115 - 119 ° C.

(2) Rotation:

The value of rotation as mesured immediately after the preparation of a 0.46% chloroform solution of the powder of MF 730-N6 is $[\alpha]_D^{26}$ = -28.5 °.

(3) Solubility:

The white powder of the antibiotic MF 730-N6 is easily soluble in methanol, ethanol, ethyl acetate and benzene, but is substantially insoluble in water or h-hexane.

(4) Color reactions:

The results of color reactions of a fraction containing the antibiotic MF 730-N6 obtained by silica gel chromatographic development of a methanol solution of the white powder prepared by the method described in Example 1 are as follows.

| | |
|---|---|
| Triphenyl tetrazolium chloride | positive |
| Anise-aldehyde - sulfuric acid reaction | positive |
| Iodine vapor | positive |
| Fehling's reagent | positive |
| Silver nitrate - ammonia (Tollens') reagent | positive |
| 8% Phosphomolybdic acid - ethanol | positive |
| Dragendorff's reagent solution | negative |
| Ferric chloride reagent solution | negative |

(5) Infrared absorption spectrum:

A white powder of antibiotic MF 730-N6 prepared by the KBr tablet method exhibits characteristic maximum absorptions at 3430, 2960, 1705 and 1635 $cm^{-1}$.

(6) $^1$H-NMR spectrum:

A white powder of MF 730-N6 immediately after its dissolution in deutero-methanol exhibits characteristic absorptions at 1.35 (s), 1.50 - 1.80 (m), 1.75 (brs), 1.80 - 2.30 (m), 2.70 - 2.85 (m) and 5.30 - 5.40 (m) in terms of δ values.

(7) Ultraviolet absorption spectrum:

A white powder of MF 730-N6 immediately after its dissolution in one of the solvent systems listed below exhibits the following maximum absoprtions and

$$E_{1\ cm}^{1\%}$$

(parenthesized) values:

methanol: 203 (60), 280 (3)

0.1N HCℓ - methanol: 203 (58), 280 (3)

0.01N NaOH - methanol: 207 (124), 267 (48).

(8) Derivatives of antibiotic MF 730-N6 and their physicochemcial properties

Experiment 1: Preparation of Wittig adduct

To a solution of 30 mg of a crude product of MF 730-N6 in 50 mℓ of anhydrous benzene, 100 mg of carbomethoxymethylene triphenyl phosphoran was added and the mixture was stirred at room temperature for 18 hours. The reaction mixture was passed through a column (15 mm x 240 mm) packed with 30 g of silica gel and the adsorbed mixture was eluted with a mixed solvent of benzene and ethyl acetate (10:1). The active fractions were collected and purified by passage through a column (20 mm x 350 mm) packed

with Sephadex LH-20 (100 mℓ), followed by elution with a mixed solvent of ethyl acetate and methanol (2:1). By these procedures, 125 mg of a 3:1 equilibrium mixture of adducts 1 and 2 was obtained.

Rf: 0.44 (benzene-acetone, 1:1)
0.40 (benzene-ethyl acetate, 3:1)

Adduct 1 (main product):

$^1H$ = NMR ($CDCℓ_3$) δ :
0.99 (3H, d, J = 7.0 Hz)
1.00 (3H, s)
1.35 (3H, s)
1.47 (1H, dd, J = 10.5 Hz, 15.0 Hz)
1.50 ~ 1.75 (3H, m)
1.76 (4H, brs)
1.95 ~ 2.10 (2H, m)
2.25 (1H, brs)
2.64 (1H, dd, J = 2.0 Hz, 12.0 Hz)
2.85 (1H, d, J = 5.0 Hz)
3.17 (1H, d, J = 5.0 Hz)
3.66 (1H, d, J = 4.0 Hz)
3.79 (3H, s)
3.80 (3H, s)
4.20 (1H, brd, J = 13.0 Hz)
4.27 (1H, dd, J = 4.0 Hz, 14.0 Hz)
5.35 (1H, br)
6.18 (1H, s)
6.38 (1H, d, J = 16.2 Hz)
8.47 (1H, d, J = 16.2 Hz)

Adduct 2 (by-product):

0.84 (3H, s)
0.89 (3H, s)
1.26 (3H, d, J = 7.0 Hz)
1.46 (1H, dd, J = 9.5 Hz, 15.0 Hz)
1.50 ~ 2.75 (4H, m)
1.81 (3H, brs)
1.95 ~ 2.10 (2H, m)
2.18 (1H, brs)
2.80 (1H, q, J = 7.0 Hz)
2.85 (1H, d, J = 5.0 Hz)
3.16 (1H, d, J = 5.0 Hz)
3.79 (3H, s)
3.81 (3H, s)
3.85 (1H, d, J = 5.0 Hz)
4.03 (1H, brd, J = 9.5 Hz)
4.38 (1H, d, J = 5.0 Hz)
5.23 (1H, br)
6.12 (1H, s)
6.37 (1H, d, J = 16.2 Hz)
8.43 (1H, d, J = 06.2 Hz)

Experiment 2: Preparation of p = Bromobenzoyl form

Ten milligrams of a mixture of adducts 1 and 2 was dissolved in 1 mℓ of anhydrous pyridine. After addition of 20 mg of p-bromobenzoyl chloride, the mixture was stirred at room temperature for 18 hours. The reaction mixture was concentrated under vacuum, and the residue was dissolved in 30 mℓ of ethyl acetate. The solution was washed with saturated aqueous solutions of sodium hydrogencarbonate and

sodium chloride, and dried over anhydrous sodium sulfate. The solvent was distilled off under vacuum. The residue was purified by passage through a column (15 mm x 105 mm) packed with 10 g of silica gel, followed by elution with a mixed solvent of benzene and ethyl acetate (20:1). By these procedures, 8.2 mg of a p-bromobenzoyl form 3 was obtained.
Rf = 0.34 (benzene-ethyl acetate, 5:1)
$[\alpha]_D^{20}$ - 66.5° (C 0.2 $CHCl_3$)

$$UV\ \lambda_{max}^{CH_3OH}\ nm\ (\varepsilon);\ 247\ (27700),\ 268\ (17100)$$

$$IR\ \nu_{max}^{CHCl_3}\ cm^{-1}\ \ 2950,\ 1720,\ 1280,\ 1175$$

FD-MS (m/z); 660, 658 ($M^+$)
HI-MS (m/z); 660, 1707 ($C_{33}H_{39}O_9Br$)
658, 1768 ($C_{33}H_{39}O_9Br$)

H-NMR ($CDCl_3$) δ :
0.88 (3H, d, J = 7.0 Hz)
1.04 (3H, s)
1.39 (3H, s)
1.55 91H, dd, J = 10.0 Hz, 15.0 Hz)
1.66 (1H, d, J = 15.0 Hz)
1.6 (1H, br)
1.75 (1H, br)
1.74 (3H, br)
2.07 (2H, br)
2.17 (1H, dq, J = 14.0 Hz, 7.0 Hz)
2.29 (1H, br)
2.84 (1H, d, J = 5.0 Hz)
3.18 (1H, d, J = 5.0 Hz)
2.91 (1H, dd, J = 1.5 Hz, 12.0 Hz)
3.75 (3H, s)
3.77 (3H, s)
4.42 (1H, brd, J = 10.0 Hz)
5.36 (1H, br)
5.81 (1H, d, J = 14.0 Hz)
6.18 (1H, s)
6.45 (1H, d, J = 16.5 Hz)
7.59 (2H, d, J = 8.5 Hz)
7.94 (2H, d, J = 8.5 Hz)
8.40 (1H, dd, J = 1.0 Hz, 16.5 Hz)

As will be apparent from the data shown above, the antibiotic MF 730-N6 of the present invention closely resembles the known substance PR-1350, but the former is a novel compound which clearly differs from the known compound in that it has 4 methyl groups and 2 hydroxyl groups.

Biological properties of antibiotic MF 730-N6

(1) Antimicrobial spectrum:

Table 1 shows the minimum concentrations of antibiotic MF 730-N6 necessary to inhibit the growth of several bacteria on a common nutrient agar plate.

Table 1

| Microorganisms | MIC (mcg/mℓ) |
|---|---|
| Staphylococcus aureus 209P | 6.25 |
| Staphylococcus aureus Smith | <0.05 |
| Micrococcus albus FDA 16 | 0.39 |
| Micrococcus lysodeikticus IFO 3333 | 0.39 |
| Sarcina lutea PCI 1001 | 0.39 |
| Bacillus anthracis | 0.2 |
| Bacillus subtilis NRRLB-558 | 0.1 |
| Bacillus subtilis PCI 219 | <0.05 |
| Bacillus cereus ATCC 10702 | 0.1 |
| Corynebacterium bovis 1810 | <0.05 |
| Escherichia coli NiHJ | 25 |
| Escherichia coli K-12 | 3.12 |
| Escherichia coli ML 1629 | 6.25 |
| Shigella dysenteriae JI 11910 | <0.05 |
| Shigella flexneri 46JS 11811 | 1.56 |
| Shigella sonnei JS 11746 | 12.5 |
| Salmonella typhi T-63 | 3.12 |
| Salmonella enteritidis 1891 | 0.1 |
| Proteus vulgaris OX19 | 12.5 |
| Proteus mirabilis IFMCM-9 | 12.5 |
| Proteus rettgeri GN311 | 12.5 |
| Proteus rettgeri GN466 | 6.25 |
| Serratia marcescens | 50 |
| Pseudomonas aeruginosa $A_3$ | 25 |
| Klebsiella pneumoniae PCI 602 | 0.39 |
| Mycobacter 607 | 0.39 |
| Xanthomonas citri | 3.12 |
| Xanthomonas oryzae | 25 |

(2) Antitumor activity

Table 2 shows the therapeutic effects of antibiotic MF 730-N6 against mouse leukemia L1210 and mouse Ehrlich's ascitic cancer (EAC).

Table 2

| Dose (mcg/mouse/day) | Percent life prolongation * | |
|---|---|---|
| | L-1210 | EAC |
| 125 | 161 | 77 |
| 62.5 | 161 | 188 |
| 31.25 | 170 | >354 |
| 15.6 | 200 | >369 |
| 7.8 | 160 | 215 |

* Percent life prolongation =

$$\frac{\text{Number of days for which treated mice survived}}{\text{Number of days for which untreated mice survived}} \times 100$$

The treated mice were those to which the antibiotic was administered intraperitoneally for continuous 10 days immediately following intraperitoneal inoculation with 1 x $10^5$ L-1210 cells per mℓ or 2 x $10^6$ EAC cells per mℓ. As Table 2 shows, significant life prolonging effects were exhibited by the antibiotic of the present invention.

(3) Acute toxicity

The acute toxicity of the antibiotic MF 720-N6 was $LD_{50}$ =100 mg/kg for intraperitoneal administration to mice.

The antibiotic MF 730-N6 characterized above can be produced with advantage by the following process in accordance with the second aspect of the present invention: An antibiotic MF 730-N6 producing microorganism is cultured on a nutrient medium and the desired antibiotic is recovered from the culture.

The microorganism used in the process of the present invention may be of any genus and species that has the ability to produce the antibiotic MF 730-N6. An advantageous example is actinomycete MF 730-N6 that is a microorganism of the genus Actinomycetes and which was isolated by the present inventors from the soil sampled in an area near Umawatashi Bridge across Iwaigawa River, Nara, Japan and which was deposited with the Fermentation Research Institute, the Agency of Industrial Science and Technology, MITI, under FERM P-No. 8247 (transmitted to FERM BP-1045) on May 21, 1985.

The microorganism may be cultured with any nutrient sources that are commonly used in cultivation of actinomycetes such as carbohydrates, nitrogen sources, inorganic salts and other assimilable sources. Usable carbon sources include carbohydrates such as glucose, glycerin, maltose, sucrose, molasses, dextrin and starch, and fats and oils such as soybean oil and peanut oil. Usable nitrogen sources include peptone, meat extract, cottonseed powder, soybean powder, yeast extract, casein, corn steep liquor, NZ-amine, ammonium sulfate, ammonium nitrate and ammonium chloride. Usable inorganic salts include dipotassium phosphate, sodium phosphate, sodium chloride, calcium carbonate, magnesium sulfate and manganese chloride. If necessary, trace metals such as cobalt and iron may be added. Any other known nutrient sources that can be utilized by the MF 730-N6 producing microorganism for production of the desired antibiotic may be used.

The proportions of the nutrient sources illustrated above are not limited to any particular values and may be varied over a broad range. The nutrient sources optimum for the growth of the antibiotic MF 730-N6 producing microorganism used and the proportions of the specific sources may be readily determined by those skilled in the art on the basis of a simple small-scale experimentation.

The nutrient medium containing the nutrient sources listed above may be sterilized prior to cultivation. It

is advantageous that the pH of the medium is adjusted to the range of 6 - 8, especially 6.5 - 7.5, either prior to or following the sterilization.

Cultivation of the antibiotic MF 730-N6 producing microorganism on the nutrient medium may be carried out in accordance with any of the methods commonly used in the production of antibiotics from actinomycetes. Cultivation under aerobic conditions is generally advantageous, and it may be accompanied by agitation and/or aeration. Stationary culture, shake culture or submerged culture with aeration and agitation may be employed, and submerged culture is suitable for mass production of the antibiotic MF 730-N6.

The cultivation temperature is not limited to any particular value so long as it is within the range that permits substantial growth of the antibiotic MF 730-N6 producing microorganism so as to ensure production of the intended antibiotic. While a suitable cultivation temperature can be selected depending upon the MF 730-N6 producing microorganism used, a particularly preferred temperature is within the range of 25 - 30 °C.

The cultivation may be continued until substantial accumulation of the intended antibiotic MF 730-N6 occurs. The cultivation time will vary with the composition of the medium, the cultivation temperature, ambient temperature or the bacterial strain used. Usually, a cultivation for 12-24 hours is sufficient for obtaining the desired antibiotic.

The amount of antibiotic MF 730-N6 accumulated in the culture may be determined with pneumobacillus strain KP PCI 606 by the cylinder-plate process conventionally employed in antibiotic determinations.

The antibiotic MF 730-N6 thus accumulated in the culture may be recovered in the isolated and pure form by subjecting the filtrate of culture or supernatant either to solvent extraction (with the aid of a suitable organic solvent) or to chromatography (by means of adsorption or ion exchange), used independently or in combination. The filtrate or supernatant of culture free from the cells may be obtained by a known separating method such as filtration or centrifugation. Organic solvents that can be used in the extraction technique include chloroform, ethyl acetate and any other solvents that are capable of dissolving the antibiotic MF 730-N6 and which are substantially insoluble in water. Examples of the chromatographic carrier having adsorptive or ion-exchanging abilities include activated carbon, silica gel- porous polystyrene-divinylbenzene resin, and many other ion-exchange resins.

By employing the procedures described above, the antibiotic MF 730-N6 having the already specified characteristics is obtained.

The following examples are provided to further illustrate the advantages of the present invention.

Example 1: Preparation of Antibiotic MF 730-N6

Actinomycetes strain MF 730-N6 (FERM P-No. 8247, transmitted to FERM BP-1045) was cultured on an agar slant medium. A liquid medium (500 mℓ, pH 7.2) containing 1% glucose and 1% yeast extract was poured into two flasks in amounts of 110 mℓ and routinely sterilized at 120 °C for 20 minutes. Each of the sterilized mediums was inoculated with the cultured cells of MF 730-N6 and subjected to rotary shake culture (180 rpm) at 27 °C for 48 hours so as to prepare a seed culture solution.

The seed culture solution (110 mℓ) was transferred into a 30-ℓ jar fermenter containing 15 liters of an MF 730-N6 producing medium (for its composition, see below) and subjected to cultivation for 30 hours at 27 °C and 150 rpm while air was supplied at a rate of 15 liters per minute. While the cultivation was continuing, a few drops of Silicone KM-75 (tradename of Shinetsu Chemical Industry Co., Ltd. for an anti-foaming agent) were applied to the medium.

A culture solution prepared in two units of the jar fermenter was suction-filtered using a filtration aid to obtain 24 liters of a culture filtrate (in the following pages, material balances are shown on the assumption that 1 mℓ of the filtrate has an antibacterial activity of 1 U against the pneumobacillus strain KP PCI 606).

The filtrate was adjusted to a pH of 5.0 and subject to two extractions with 12 liters of chloroform (total activity of the extract: 15,000 U/T).

The extract was concentrated to a volume of 1 liter under vacuum. The concentrate was dehydrated with Glauber's salt and after filtration, discolored with 0.5% activated carbon. After filtering off the activated carbon, the filtrate was concentrated under vacuum. The concentrate was subjected to silica gel chromatography (3.5 cm x 11 cm), followed by elution with a mixed solvent of chloroform/methanol (100/3). The collected active fractions were concentrated under vacuum, and the concentrate was subjected to column chromatography (3.5 cm x 11 cm) using Diaion HP-20 (trademark of Mitsubishi Chemical Industries, Limited) and eluted by a linear density gradient of 50% aqueous methanol to 100% methanol. The active fractions were collected, freed of methanol under vacuum, and freeze-dried to obtain 108mg of a pale yellow powder (6,500 U/T).

This powder was subjected to reverse-phase TLC developed with a mixed solvent of acetonitrile/water (7/3). The collected active fractions were subjected to extraction with methanol to obtain 39mg (5460 U/T) of the pure form of antibiotic MF 730-N6. This material is characterized by the physicochemical properties already described.

**Composition of MF 730-N6 Producing Medium**

| | |
|---|---|
| Glucose | 0.08 (%) |
| Galactose | 0.16 |
| Maltose | 0.16 |
| Dextrin | 0.32 |
| Bactosoytone | 0.16 (%) |
| Ammonium sulfate | 0.16 |
| Antifoaming agent | 1 or 2 drops |
| | pH 7.2 (PR) |

Mycological Properties:

The strain MF 730-N6 (FERM P-No. 8247, transmitted to FERM BP-1045) used in Example 1 has the following mycological properties.

1. Morphology

Strain MF 730-N6 as observed under a microscope has relatively long, straight (restiflexibiles) aerial mycelia extending from branched aerial hyphae, but no spiral for motion or whirl is observed. Mature spore chains have a detectable chain of not less than 10 to 20 spores. The spore size is in the range of 0.4 - 0.6 x 0.8 - 1.4 µm. The spores are smooth-surfaced, but SEM observation reveals distinct "wrinkles" on the spore surface.

2. State of growth on media

In the following description, color standards are bracketed and they are in accordance with Color Harmony Manual of Container Corporation of America.

(1) Sucrose-nitrate agar medium (incubated at 27° C)

Adhering light brown gray [3dc, Natural - 3fe, Silver Gray] aerial mycelia form on a colorless growth. No soluble dye observed.

(2) Glucose-asparagine agar medium (27° C)

Adhering light brown gray [3fe, Silver Gray] aerial form on a pale yellow brown [2ec, Oatmeal] growth. No soluble dye observed.

(3) Glycerin-asparagine agar medium (ISO-medium 5, incubated at 27° C)

Adhering light brown gray [3dc, Natural] aerial mycelia form on a colorless growth. No soluble dye observed.

(4) Starch-inorganic salt agar medium (ISP-medium 4, incubated at 27° C)

Adhering light brown gray [3fe, Silver Gray - 5fe, Ashes] aerial mycelia form on a pale yellow brown [3gc, Bamboo] growth. No soluble dye observed.

(5) Tyrosine agar medium [ISP-medium 7, incubated at 27° C)

Adhering light brown gray [5fe, Ashes] are mycelia form on a colorless growth. No soluble dye observed.

(6) Nutrient agar medium (incubated at 27° C)

Pale yellow brown [2gc, Bamboo] growth. No adhering aerial mycelia. No soluble dye observed.

(7) Yeast-malt agar medium (ISP-medium 2, incubated at 27° C)

Adhering light brown gray [3fe, Silver Gray] aerial mycelia form on a pale yellow brown [2ie, Lt Mustard Tan] growth. No soluble dye observed.

(8) Oatmeal agar medium (ISP-medium 3, incubated at 27° C)

Adhering light brown gray [5fe, Ashes] aerial mycelia form on a colorless growth. No soluble dye observed.

(9) Glycerin-nitrate agar medium (incubated at 27° C)

Adhering light brown gray [3dc, Natural - 3fe, Silver Gray] aerial mycelia form on a colorless growth. No soluble dye observed.

(10) Starch agar medium (incubated at 27° C)

A thin layer of adhering white aerial mycelia forms on a pale yellow [3ca, Shell] growth. No soluble dye observed.

(11) Calcium malate agar medium (incubated at 27° C)

A thin layer of adhering white aerial mycelia forms on a colorless to pale yellow [2ca, Lt Ivory] growth. No soluble dye observed.

(12) Cellulose (incubated at 27° C in a synthetic solution containing filter paper chips)

Colorless growth and light brown gray aerial mycelia. No soluble dye observed.

(13) Gelatin stab culture

In both incubation on a simple gelatin medium at 20° C and incubation on a glucose-peptone gelatin medium at 27° C, the growth is colorless and no adhering aerial mycelia forms. No soluble dye observed.

(14) Skimmed cow's milk (incubated at 37° C)

Colorless growth and no adhering aerial mycelia. No soluble dye observed.

## 3. Physiological properties

(1) Growth temperature range

Growth test was conducted on glucose-asparagine agar mediums at varying temperatures, 20° C, 24° C, 27° C, 30° C, 37° C and 50° C. Except at 50° C, the microorganism grew at each of the temperatures tested, but an optimal temperature would be in the range of 27 - 30° C.

(2) Liquefaction of gelatin (on 15% simple gelatin medium at 20° C, or on glucose-peptone gelatin medium at 27° C)

With the simple gelatin medium, liquefaction started at about 5 days of incubation and the degree of liquefaction was moderate to strong.

With the glucose-peptone gelatin medium, liquefaction started at about 2 days of incubation and the degree of liquefaction was rather strong.

(3) Hydrolysis of starch (incubated at 27° C on both a starch-inorganic salt agar medium and a starch agar medium)

Hydrolysis of starch was observed on each medium starting at about 5 days of incubation, and the degree of hydrolysis was moderate.

(4) Coagulation and peptonization of skimmed cow's milk (incubated on skimmed cow's milk at 37° C)

Coagulation of skimmed cow's milk started at about 2 days of incubation and was soon completed, followed by peptonization, which was completed on the 15th day of incubation. The degree of coagulation and peptonization was rather strong.

(5) Formation of melanine-like pigment (with tryptone-yeast broth, ISP-medium 1; peptone-yeast-iron agar, ISP-medium 6; or tyrosine agar, ISP-medium 7, at 27° C)

No melanine-like pigment observed in either medium.

(6) Utilization of carbon sources (on Pridham-Gottlieb agar medium, ISP-medium 9, at 27° C)

The microorganism grew utilizing D-glucose, D-xylose, and L-arabinose, but not utilizing D-fructose, sucrose, inositol, rhamnose or D-mannitol. The microorganism would probably not utilize raffinose.

(7) Dissolution of calcium malate (on calcium malate agar at 27° C)

No dissolution of calcium malate observed.

(8) Reduction of ntirate (in aqueous solution of peptone containing 0.1% $KNO_3$, ISP-medium 8, at 27° C)

Negative.

## 4. Chemical properties

(1) 2,6-diaminopimelic acid, DAP

Meso-2,6-diaminopimelic acid and a small portion of LL-2,6-diaminopimelic acid were observed in the whole cell and the cell wall.

Aerial mycelium fractions were obtained by the method of Ohmura et al. (Journal of Antibiotics, 34, 1633, 1981) and they contained a major amount of LL-2,6-diaminopimelic acid and a minor portion of meso-2,6-diaminopimelic acid. Aerial hypha fractions were also obtained by the same method and they were found to contain a major amount of meso-2,6-diaminopimelic acid, with a trace amount of the LL-form.

(2) Sugars

Mannose, galactose, glucose and ribose were found in the whole cell, while mannose and galactose were observed in the cell wall. Each of the whole cell and the cell wall produced a small amount of unidentifiable spots in a color reaction test.

(3) Content of GC (guanine and cytosine) in DNA (deoxyribonucleic acid)

A GC content of 70.8% was obtained by the UV absorption method described in S. Ulitzur, Biochimica et Biophysica Acta, 272, 1972.

(4) LCN-A (lipid characteristic Nocardia - A)

No LCN-A was observed upon analysis by the TLC technique described in Mordarska, H. and M. Mordarski: Journal of General Microbiology, 71, 1972.

(5) Acyl type of amino sugars in cell wall

The amino sugars in the cell were found to be of the acetyl type by the method of Uchida et al. described in Kinya Uchida and Kō Aida: Journal of General Applied Microbiology, 23, 249 - 260, 1977.

(6) Glycine in cell wall

Glycine was observed in the cell wall.

The above observations can be summarized as follows: morphologically, the aerial mycelia of the strain MF 730-N6 have neither spiral formation nor whirls. A mature spore chain has at least 10 - 20 spores. The organism has smooth-surfaced spores. It grows on a variety of media, abundantly providing light brown gray aerial mycelia on colorless to pale yellow brown growths. No soluble dye observed. No melanine-like pigment formed. The organism has strong ability to decompose protein. The degree of starch hydrolysis is moderate.

Meso- and LL-2,6-diaminopimelic acid, as well as glycine and galactose are observed in the cell wall of the strain MF 730-N6. The predominant portion of the 2,6-diaminopimelic acid in aerial mycelia is the LL-form while that in aerial hyphae fractions is the meso-form. The acryl type of the amino sugars in the cell wall is of acetyl type. No LCN-A observed. The GC content of DNA is 70.8%.

On the basis of these observations, the present inventors confirmed that the strain MF 730-N6 belongs to none of the Types I to IX proposed by Lechevalier et al. (International Journal of Systematic Bacteriology, 20, 435, 1970) for classification of the predominant components of cell walls.

The strain MF 730-N6 exhibits properties close to those of microorganisms of the genus Kitasatosporia established by Ohmura et al. in 1982 (The Journal of Antibiotics, 35, 1013, 1982, followed up by Bulletin of Nippon Hosenkin Kenkyukai, No. 45, December, 1984). In particular, the fact that this strain formed morphologically distinct submerged spores in a liquid culture solution presented a strong reason for the inventors to classify the strain as falling within the genus Kitasatosporia. Of the known four species of Kitasatosporia, Kitasatosporia setae exhibits properties most similar to those of the strain MF 730-N6 [see Ohmura et al., ibid, and "Hosenkin no Dotei Jikkenho (Experimental Methods for Identification of Actinomycetes)", ed. by Nippon Hosenkin Kenkyukai, 1985, p. 246].

The strain Kitasatosporia setaruba (currently named satae) was cultured for comparison with MF 730-N6. The results are shown in Table 3.

Table 3

| | MF 730-N6 | Kitasatosporia setaruba IMC-A-0122 (IFO 14126) Experimental data | Date in the literature |
|---|---|---|---|
| Spore surface | smooth (with "wrinkles") | smooth (with "wrinkles") | smooth (with "wrinkles") |
| Form of aerial mycelia | straight | straight | straight |
| Color of aerial mycelia | light brown gray | white - brownish white - light brown gray | white - light gray |
| Spore chain | ≥ 10 - 20 spores | ≥ 10 - 20 spores | ≥ 20 spores |
| Color of growth | colorless - pale yellow brown | colorless - pale yellow brown | colorless - pale yellow brown |
| Soluble dye | - ∿ pale yellow | - ∿ pale yellow | - ∿ pale yellow |
| Formation of melanine-like pigment | | | |
| ISP-medium 1 | - | - | |
| ISP-medium 6 | - | - | |
| ISP-medium 7 | - | - | |
| Hydrolysis of starch | + | + | + |
| Coagulation and peptonization of cow's milk | + strong | + strong | + |
| Liquefaction of gelatin | + strong | + strong | - |
| Reduction of nitrate | - | + | - |
| Utilization of carbon sources | | | |
| D-glucose | + | + | + |
| L-arabinose | + | + | + |
| D-xylose | + | + | + |
| D-fructose | - | - | - |
| Sucrose | - | - | - |
| L-rhamnose | - | - | - |
| Raffinose | ∓ | - | - |
| D-mannitol | - | - | - |
| Growth temperature range | 20 - 37°C | 20 - 37°C | 22 - 37°C |
| Optimal temperature | 27 - 30°C | 24 - 30°C | |
| 2,6-diaminopimelic acid sugar in the whole cell | meso- and LL-forms, predominantly galactose, mannose and ribose | meso- and LL-forms, predominantly galactose, mannose and ribose | meso- and LL-forms, glactose (xylose) |
| 2,6-diaminopimelic acid sugar in the whole cell | meso- and LL-forms, galactose and mannose | meso- and LL-forms, galactose and mannose | meso- and LL-forms, galactose |
| Glycine | + | + | + |
| Acyl type of amino sugar | acetyl type | acetyl type | acetyl type |
| 2,6-diaminopimelic acid sugar in acrial mycelia | predominantly LL-form | predominantly LL-form | LL-form |
| 2,6-diaminopimelic acid sugar in aerial hyphae | predominantly meso-form | predominantly meso-form | meso-form |
| GC content of DNA | 70.8% | | 73.1% |
| LCN-A | - | - | |
| Antibiotic produced | Terpenticin | | Setamycin |

∓ : probably

As the table shows, the strain MF 730-N6 is very similar to Kitasatosporia setae, with the only differences existing in terms of the reduction of a nitrate, GC content of CNA, sugar components (rhamnose and ribose) of the whole cell. Of these four differences, the one concerning the reduction of a nitrate is not considered to be significant in view of the data in the literature. The significance of the difference in terms of the GC content of DNA is difficult to evaluate because it largely depends on the method of measurement.

As for the the sugar contents of the whole cell, the strain MF 730-N6 was found to contain a small amount of rhamnose and Kitasatosporia setaruba contained a very small amount of ribose. Therefore, the strain MF 730-N6 is assumed to be a species that is closely homologous to Kitasatosporia setae (formerly setaruba).

## Claims

**Claims for the following Contracting States : BE, CH, DE, FR, GB, IT, LI, LU, NL, SE**

**1.** The compound MF 730-N6, which is characterized by the following properties:
A) melting point:
115 - 119 °C
B) rotation as measured immediately after preparation of a solution of the antibiotic:
$[\alpha]_D^{26}$ = -28.5 ° (C = 0.46, chloroform)
C) solubility:
soluble in methanol, ethanol, ethyl acetate and benzene
D) color reactions:

| | |
|---|---|
| triphenyl tetrazolium chloride reaction | positive |
| anise-aldehyde - sulfuric acid reaction | positive |
| iodine vapor | positive |
| Fehling's reagent | positive |
| silver nitrate - ammonia (Tollens') reagent | positive |
| 8% phosphomolybdic acid - ethanol | positive |
| Dragendorff's solution | negative |
| ferric chloride reagent solution | negative |

E) ultraviolet absorption spectrum:

$$\lambda_{max}^{CH_3OH} (E_{1\,cm}^{1\%}) = 203\ nm\ (60),\ 280\ nm\ (3)$$

F) infrared absorption spectrum:
distinct characteristic absorption maxima appearing at wave numbers of 3430, 2960, 1705 and 1635 $cm^{-1}$ by the KBr tablet method; and
G) $^1H$ - NMR spectrum ($CH_3OD$):
$\delta$ = 1.35 (s), 1.50 - 1.80 (m), 1.75 (brs), 1.80 - 2.30 (m), 2.70 - 2.85 (m), and 5.30 - 5.40 (m).

**2.** A process for the preparation of the compound as defined in claim 1, which comprises cultivating a microorganismus of the genus Kitasatosporia capable of producing the compound MF 730-N6 on a nutrient medium and recovering said compound from the culture by usual methods of solvent extraction and/or chromatography.

**3.** Use of a compound as defined in claim 1 for the preparation of a medicament having anti-tumor and antimicrobial activities.

**4.** The strain Kitasatosporia setae MF 730-N6 (FERM p-No. 8247 transmitted to FERM BP-1045).

**Claims for the following Contracting State : AT**

1. A process for the preparation of the compound MF 780-N6 characterized by the following properties:
   A) melting point:
   115 - 119°C
   B) rotation as measured immediately after preparation of a solution of the antibiotic:
   $[\alpha]_D^{20}$ = -28.5° (C = 0.46, chloroform)
   C) solubility:
   soluble in methanol, ethanol, ethyl acetate and benzene
   D) color reactions:

| | |
|---|---|
| triphenyl tetrazolium chloride reaction | positive |
| anise-aldehyde - sulfuric acid reaction | positive |
| iodine vapor | positive |
| Fehling's reagent | positive |
| silver nitrate - ammonia (Tollens') reagent | positive |
| 8% phosphomolybdic acid - ethanol | positive |
| Dragendorff's solution | negative |
| ferric chloride reagent solution | negative |

   E) ultraviolet absorption spectrum:

$$\lambda_{max}^{CH_3OH} (E_{1\ cm}^{1\%}) = 203\ nm\ (60),\ 280\ nm\ (3)$$

   F) infrared absorption spectrum:
   distinct characteristic absorption maxima appearing at wave numbers of 3430, 2960, 1705 and 1635 $cm^{-1}$ by the KBr tablet method; and
   G) $^1H$ - NMR spectrum ($CH_3OD$):
   $\delta$ = 1.35 (s), 1.50 - 1.80 (m), 1.75 (brs), 1.80 - 2.30 (m), 2.70 - 2.85 (m), and 5.30 - 5.40 (m)

   which comprises cultivating a microorganism strain of the genus Kitasatosporia capable of producing the compound MF 730-N6 in a nutrient medium consisting of carbon sources, nitrogen sources, inorganic salts and optionally trace metals and recovering the produced compound by subjecting the filtrate of the culture broth or the supernatant to solvent extraction and/or to chromatography.

2. The process as defined in claim 1 wherein the strain is cultivated at a pH of from 6 to 8, a temperature of from 25 to 70°C and a cultivation time of from 12 to 24 hours.

3. The process as defined in claim 1 wherein the chromatography is conducted with adsorptive or ion-exchanging abilities.

4. The process as defined in claim 1 where the microorganism strain used is Kitasatosporia setae MF 730-N6 (FERM P-No. 8247 transmitted to FERM BP-1045).

**Patentansprüche**
**Patentansprüche für folgende Vertragsstaaten : BE, CH, DE, FR, GB, IT, LI, LU, NL, SE**

1. Verbindung MF 730-N6, gekennzeichnet durch folgende Eigenschaften:
   (A) Schmelzpunkt:
   115 - 119°C

(B) Drehung, gemessen unmittelbar nach Herstellung einer Lösung des Antibiotikums:
$[\alpha]_D^{26}$ = -28.5° (C = 0.46, Chloroform)
(C) Löslichkeit:
löslich in Methanol, Ethanol, Ethylacetat und Benzol
(D) Farbreaktionen:

| | |
|---|---|
| Triphenyltetrazoliumchlorid-Reaktion | positiv |
| Anisaldehyd-Schwefelsäure-Reaktion | positiv |
| Joddampf | positiv |
| Fehling's Reagens | positiv |
| Silbernitrat-Ammoniak (Tollens) Reagens | positiv |
| 8 % Phosphormolybdänsäure-Ethanol | positiv |
| Dragendorff's Lösung | negativ |
| Eisen(III)-chlorid-Reagenslösung | negativ |

(E) Ultraviolett-Absorptionsspektrum:

$$\lambda_{max}^{CH_3OH} (E_{1\,cm}^{1\%}) = 203\ nm\ (60),\ 280\ nm\ (3)$$

(F) Infrarot-Absorptionsspektrum:
klare, charakteristische Absorptionsmaxima bei Wellenlängen 3430, 2960, 1705 und 1635 $cm^{-1}$, ermittelt durch das KBr-Tablettenverfahren, und
(G) $^1H$ - NMR Spektrum ($CH_3OD$):
$\delta$ = 1.35 (s , 1.50 - 1.80 (m), 1.75 (brs), 1.80 - 2.30 (m), 2.70 - 2.85 (m), und 5.30 - 5.40 (m).

**2.** Verfahren zur Herstellung der Verbindung nach Anspruch 1, umfassend die Kultivierung eines zur Produktion der Verbindung MF 730-M6 fähigen Mikroorganismusstamm der Art Kitasatosporia in einem Nährmedium und die Sammlung der Verbindung aus der Kultur mittels üblicher Lösungsmittelextraktions- und/oder Chromatographie-Verfahren.

**3.** Verwendung einer Verbindung nach Anspruch 1 zur Herstellung eines Medikaments mit Antitumor und antimikrobieller Aktivität.

**4.** Stamm Kitasatosporia setae MF 730-M6 (FERM p-Nr. 8247, übergeben an FERM BP-1045).

**Patentansprüche für folgenden Vertragsstaat : AT**

**1.** Verfahren zur Herstellung der durch die folgenden Eigenschaften gekennzeichneten Verbindung MF 730-M6:
(A) Schmelzpunkt:
115 - 119°C
(B) Drehung, gemessen unmittelbar nach Herstellung einer Lösung des Antibiotikums:
$[\alpha]_D^{26}$ = -28.5° (C = 0.46, Chloroforn)
(C) Löslichkeit:
löslich in Methanol, Ethanol, Ethylacetat und Benzol
(D) Farbreaktionen:

| | |
|---|---|
| Triphenyltetrazoliumchlorid-Reaktion | positiv |
| Anisaldehyd-Schwefelsäure-Reaktion | positiv |
| Joddampf | positiv |
| Fehling's Reagens | positiv |
| Silbernitrat-Ammoniak (Tollens) Reagens | positiv |
| 8 % Phosphormolybdänsäure-Ethanol | positiv |
| Dragendorff's Lösung | negativ |
| Eisen(III)-chlorid-Reagenslösung | negativ |

(E) Ultraviolett-Absorptionsspektrum:

$$\lambda^{CH_3OH}_{max} (E^{1\%}_{1\,cm}) = 203\ nm\ (60),\ 280\ nm\ (3)$$

(F) Infrarot-Absorptionsspektrum:
klare, charakteristische Absorptionsmaxima bei den Wellenlängen 3430, 2960, 1705 und 1635 $cm^{-1}$, ermittelt durch das KBr-Tablettenverfahren, und
(G) $^1H$ - NMR Spektrum ($CH_3OD$):
$\delta$ = 1.35 (s), 1.50 - 1.80 (m), 1.75 (brs), 1.80 - 2.30 (m), 2.70 - 2.85 (m), und 5.30 - 5.40 (m),
bei dem ein zur Produktion der Verbindung MF 730-M6 fähiger Mikroorganismusstamm der Art Kitasatosporia in einem Kohlenstoffquellen, Stickstoffquellen, anorganische Salze und gegebenenfalls Spurenmetalle enthaltenden Nährmedium kultiviert und die produzierte Verbindung gesammelt werden, indem das Filtrat der Kulturbrühe oder der Überstand einer Lösungsmittelextraktion und/oder Chromatographie unterzogen wird.

**2.** Verfahren nach Anspruch 1, worin der Stamm bei pH 6-8, einer Temperatur von 25-70 °C und einer Kultivierungszeit von 12-24 Stunden kultiviert wird.

**3.** Verfahren nach Anspruch 1, worin die Chromatographie mit Adsorptions- oder Ionenaustauscher-Vermögen durchgeführt wird.

**4.** Verfahren nach Anspruch 1, worin der verwendete Mikroorganismusstamm Kitasatosporia setae MF 730-M6 (FERM p-Mr. 8247, übergeben an FERM BP-1045) ist.

**Revendications**
**Revendications pour les Etats contractants suivants : BE, CH, DE, FR, GB, IT, LI, LU, NL, SE**

**1.** Composé MF 730-N6, caractérisé par les propriétés suivantes :
A) Point de fusion :
115 - 119 °C.
B) Rotation mesurée directement après la préparation d'une solution de l'antibiotique :
$[\alpha]^{26}_D$ = - 28,5 ° (C = 0,46, chloroforme)
C) Solubilité :
Soluble dans le méthanol, l'éthanol, l'acétate d'éthyle et le benzène.
D) Réactions colorées :

| | |
|---|---|
| Réaction au chlorure de triphényltétrazolium | positive |
| Réaction à l'anisaldéhyde - acide sulfurique | positive |
| Vapeur d'iode | positive |
| Réactif de Fehling | positive |
| Réactif de nitrate d'argent - ammoniaque (réactif de Tollens) | positive |
| Acide phosphomolybdique - éthanol | positive |
| Solution de Dragendorff | négative |
| Solution de réactif au chlorure ferrique | négative. |

E) Spectre d'absorption des rayons ultraviolets :

$$\lambda_{max}^{CH_3OH} (E_{1\,cm}^{1\%}) = 203 \text{ nm } (60), 280 \text{ nm } (3)$$

F) Spectre d'absorption des rayons infrarouges :

Absorption maximale caractéristique distincte apparaissant aux longueurs d'onde de 3430, 2960, 1705 et 1635 $cm^{-1}$ au moyen du procédé à pastille de KBr ; et

G) Spectre de $^1$H-RMN ($CH_3OD$) :

$\delta$ = 1,35 (s), 1,50-1,80 (m), 1,75 (s large), 1,80-2,30 (m), 2,70-2,85 (m) et 5,30-5,40 (m).

**2.** Procédé de préparation du composé tel qu'il est défini dans la revendication 1, comprenant la culture d'un micro-organisme de l'espèce *Kitasatosporia* capable de produire le composé MF 730-N6 sur un milieu nutritif et récolte dudit composé dans la culture au moyen de procédés usuels d'extraction par solvant et/ou chromatographie.

**3.** Utilisation d'un composé tel qu'il est défini dans la revendication 1 pour la préparation d'un médicament ayant une activité antitumorale et antimicrobienne.

**4.** Souche *Kitasatosporia setae* MF 730-N6 (FERM p-n° 8247 transmis à FERM BP-1045).

**Revendications pour l'Etat contractant suivant : AT**

**1.** Procédé de préparation du composé MF 780-N6 caractérisé par les propriétés suivantes :

A) Point de fusion :

115 - 119 °C.

B) Rotation mesurée directement après la préparation d une solution de l'antibiotique :

$[\alpha]_D^{26}$ = - 28,5 ° (C = 0,46, chloroforme)

C) Solubilité :

Soluble dans le méthanol, l'éthanol, l'acétate d'éthyle et le benzène.

D) Réactions colorées :

| | |
|---|---|
| Réaction au chlorure de triphényltétrazolium | positive |
| Réaction à l'anisaldéhyde - acide sulfurique | positive |
| Vapeur d'iode | positive |
| Réactif de Fehling | positive |
| Réactif de nitrate d'argent - ammoniaque (réactif de Tollens) | positive |
| Acide phosphomolybdique - éthanol | positive |
| Solution de Dragendorff | négative |
| Solution de réactif au chlorure ferrique | négative. |

E) Spectre d'absorption des rayons ultraviolets :

$$\lambda_{max}^{CH_3OH} (E_{1\,cm}^{1\%}) = 203 \text{ nm } (60), 280 \text{ nm } (3)$$

F) Spectre d'absorption des rayons infrarouges :

Absorption maximale caractéristique distincte apparaissant aux longueurs d'onde de 3430, 2960, 1705 et 1635 $cm^{-1}$ au moyen du procédé à pastille de KBr ; et

G) Spectre de $^1$H-RMN ($CH_3OD$) :

$\delta$ = 1,35 (s), 1,50-1,80 (m), 1,75 (s large), 1,80-2,30 (m), 2,70-2,85 (m) et 5,30-5,40 (m),

procédé qui comprend la culture d'une souche de micro-organisme de l'espèce *Kitasatosporia* capable de produire le composé MF 730-N6 dans un milieu nutritif formé de sources carbonées, de sources azotées, de sels inorganiques et éventuellement d'oligo-éléments, et la récolte dudit composé produit, en soumettant le filtrat du bouillon de culture ou le surnageant à une extraction au moyen d'un solvant et/ou à une chromatographie.

**2.** Procédé tel qu'il est défini dans la revendication 1, dans lequel la souche est cultivée à un pH de 6 à 8, à une température de 25 à 70 ° C et pendant un temps de culture de 12 à 24 heures.

**3.** Procédé tel qu'il est défini dans la revendication 1, dans lequel la chromatographie est effectuée avec possibilités d'absorption ou d'échange d'ions.

**4.** Procédé tel qu'il est défini dans la revendication 1, dans lequel la souche de micro-organisme utilisée est *Kitasatosporia setae* MF 730-N6 (FERM p-n° 8247 transmis à FERM BP-1045).